# EUROPEAN PATENT SPECIFICATION

(11) **EP 2 419 220 B1**
(45) Date of publication and mention of the grant of the patent: **06.06.2018**
(21) Application number: 10713296.1
(22) Date of filing: 09.04.2010
(51) Int. Cl.: B01L 9/00

(54) **PROTECTION OF BIOANALYTICAL SAMPLE CHAMBERS**
SCHUTZ VON KAMMERN FÜR BIOLOGISCHE PROBEN
PROTECTION DE CHAMBRES À ÉCHANTILLONS BIOANALYTIQUES

(30) Priority: 15.04.2009 EP 09157972
(43) Date of publication of application: 22.02.2012
(60) Divisional application: 18160211.1
(73) Proprietor: Biocartis NV, 2800 Mechelen (BE)
(72) Inventor: DE GIER, Ronald, NL-5656 AE Eindhoven (NL); VERSLEEGERS, Jozef, C., M., NL-5656 AE Eindhoven (NL)
(74) Representative: Toleti, Martin
(86) International application number: PCT/CH2010/000095
(87) International publication number: WO 2010/118542

(56) References cited:
- JP-A- 10 096 725
- US-A- 5 846 487

## Description

### FIELD OF THE INVENTION

This invention relates to apparatus for performing bioanalytic processing and analysis. In particular, the present invention relates to a bioanalytical reaction device and a cartridge thereof. The cartridge contains at least one sample chamber for storing biological samples, the bioanalytical reaction device can process and analyze.

### BACKGROUND OF THE INVENTION

One example of a bioanalytical reaction is the DNA polymerase chain reaction. The polymerase chain reaction (PCR) is a technique that permits amplification and detection of nucleic acid sequences. This technique has a wide variety of applications including DNA sequence analysis, detection of genetic mutations, diagnoses of viral infections, to name but a few. With the PCR a specific target sequence or strand of DNA can exponentially be amplificated. The polymerase chain reaction comprises repeated cycles of target denaturation by heating the sample, primer annealing at a lower temperature and polymerase-mediated extension at a slight higher temperature. At the last step, the DNA polymerase synthesizes a new DNA strand complementary to the DNA template strand. Under optimal conditions, the amount of DNA target strands is doubled.

Besides to PCR, other bioanalytical reactions are known, for example the ligase chain reaction. More generally, several import bioanalytical methods are dependent upon changing the temperature of samples in a controlled fashion. Therefore, there is a need for the automation of these methods.

Several mechanical and automated bioanalytical reaction devices are known in the art. Certain devices use cartridges for storing biological samples, so that the one or more biological samples in one cartridge can be temporarily stored, while the biological samples in another cartridge can be processed in the bioanalytical reaction device. An operator only needs to remove the one cartridge from the device and insert the other cartridge into the device.

Such cartridges have various interfaces, such as one or more interfaces for heating a sample in the cartridge as well as one or more interfaces for optical reading out the result of the reaction, which is, for example, indicated by a certain color of the sample or by certain illuminating substances.

More specifically, the samples to be processed are stored in one or more chambers in the cartridge. In general, an interface is provided by a wall of one of the chambers through which the sample can be heated or analyzed. If an optical readout has to be performed, the chamber needs a transparent wall as interface.

According to US 5,846,487 a specimen cartridge for biological materials is formed from unbreakable materials, such as cardboard or synthetic polymers. The specimen cartridge includes a handle, a receptor attached to the handle, and an outer housing. The receptor and handle are slidable relative to the outer housing which may include a bar-coding label and a security tape. The outer housing and receptor fit snugly together to provide a liquid-tight, leak-proof seal to prevent the escape of any biological materials from the receptor. Thus, the specimen cartridge minimizes the risk of contamination caused by the storage and transport of potentially hazardous biological materials. Further, the outer housing and receptor are formed of shatter-proof materials, also to prevent contamination. A carrying case is adapted for safely and securely transporting and testing a plurality of specimen cartridges.

In JP 10 096725 a device is provided with a rotating retainer which holds narrow sheeted pieces by 180 degrees in relation to a grip. Appropriate length of the grip makes handling of the device very easy and its inspection very sanitary. When an inspected solution penetrates into a membrane due to capillary action, specific components in the inspected solution uniquely combine with a movable indicating component. Then, the inspected solution combined with the movable indicating component is observed for color change in a first junction component position through a judging window and an existence of specific components or their quantity ratio is detected.

### SUMMARY OF THE INVENTION

It may be a problem, that such interfaces can be damaged or become dirty. Especially, when an operator handles such a cartridge, there is the possibility that he touches the cartridge at a location of an interface. An interface in the form of a thin wall can already be damaged by the force applied by a finger. Also, sweat or grease can be deposited on the interface in this way. A damaged or dirty interface can result in leakage from the cartridge or falsification of the optical detection.

It is an object of the invention to provide a save and simple cartridge.

According to the invention, a cartridge for a bioanalytical reaction device is provided, the cartridge comprising at least one sample chamber for a sample, the at least one sample chamber having a wall through which the sample can be processed or analyzed by the bioanalytical reaction device, wherein the cartridge comprises a housing and a platform, the platform comprising the at least one sample chamber, wherein the platform is movably connected to the housing, such that the platform is movable between a stowed position, in which the wall is protected by the housing, and an extended position, in which the wall is outside of the housing and further as defined in claim 1.

Such a cartridge is protected from becoming damaged or polluted without unnecessarily complicating the structural design of the cartridge and the bioanalytical reaction device.

It is to be understood that herein the term "cartridge" is used for every kind of device capable of being connected with a bioanalytical reaction device. For example, a cartridge may be a holder, magazine, cassette or carrier.

The at least one sample chamber is placed on a platform (or disc or carrier) that can be extended from the cartridge. In the stowed position, the sample chamber is inside the housing of the cartridge. Consequently, the chamber is protected from getting damaged or dirty. For use, the platform is extended from the cartridge, e. g. for enabling it to interface with heaters and optical sensors of a bioanalytical reaction device.

The wall of the at least one sample camber can be a heating interface or, if the wall is translucent (at least for some wavelength), an optical interface for interfacing with components of the bioanalytical reaction device, such as a heater or an optical sensor.

According to the invention, a cartridge is provided, wherein the at least one sample chamber is connected to a channel for filling the at least one sample chamber, the channel ending in the vicinity of the actuation means.

Vicinity may be understood as relating to a length of one of the following intervals: [0, 15mm], [0, 10mm], [0, 5mm].

The at least one sample chamber is connected to a channel for filling and draining the at least one sample chamber with fluids, such as the solution in which the sample is dissolved. Instead of a channel, every means adapted to conduct a fluid from one point to another, such as a line, a pipe or a hose, can be used. One end of the channel can be connected to a line of the bioanalytical reaction device, which can pump fluids over the line into the sample chamber. The end of the channel is part a fluidal interface of the cartridge.

Placing the end of the channel in the vicinity of the actuation means has the advantage that a mechanical connection for moving the platform and a fluidal connection can be integrated in one component of the cartridge.

According to the invention, a cartridge is provided, wherein a part of the channel is located within the actuation means. The channel may be located in a shaft for rotating the platform or in a spindle for moving the platform. This is one possibility of integrating the mechanical and the fluidal connection of the cartridge. Further the at least one sample chamber may be filled independent of the position of the platform.

According to a further exemplary embodiment, a cartridge is provided, wherein the wall is arranged at a first side of the platform, wherein the platform has a second side opposite to the first side, and wherein the platform in the extended position is accessible from the first side and the second side by the bioanalytical reaction device for processing or analyzing the sample. The sample within the at least sample chamber may be processed or analyzed simultaneously from two sides of the platform.

According to a further exemplary embodiment, a cartridge is provided, wherein at least one dimension of the cartridge with the platform in the extended position is bigger than this dimension of the cartridge with the platform in the stowed position. Therefore, the cartridge with the platform in the stowed position can easily be stored.

According to a further exemplary embodiment, a cartridge is provided, wherein the platform is rotatably connected to the housing. Preferably, the actuation means is a shaft and the platform is connected to the shaft for rotating the platform about a rotation axis. More preferably, the shaft extends up to an opening in the housing. In this way, the mechanical connection of an actuator of the bioanalytical reaction device to the cartridge for rotating the platform can easily be established. Further, the opening in the housing may provide a guidance for the shaft, and therefore for the platform.

Alternatively, according to a further exemplary embodiment, a cartridge is provided, wherein the platform is slidably connected to the housing. The actuation means may be a spindle for translatorily moving the platform from the stowed position to the extended position.

According to a further exemplary embodiment, a cartridge is provided, wherein the platform has the form of a plate, which, in the stowed position, is arranged between a first wall and a second wall of the housing. A platform in the form of a plate, i. e. a component with one dimension much smaller than the two other dimensions in different directions, can be provided with more than one sample chamber and all of the sample chambers are easily accessible by a bioanalytical reaction device.

According to a further exemplary embodiment, a cartridge is provided, wherein the wall of the at least one sample chamber is thin. For minimizing the thermal barrier, the wall may be thin and can for example be a foil with a high heat conductance. Herein, with a thin wall a wall is meant which has a thickness of about less than 200 µm. A thin wall may also optimize the transparence of the optical interface of the at least one sample chamber.

According to a further exemplary embodiment, a cartridge is provided, wherein the at least one sample chamber is formed by an opening in the platform which is covered by a foil or thin layer forming the thin wall.

Another aspect of the invention is a bioanalytical reaction device having a slot or receptacle for receiving the cartridge, comprising an actuator for extending and stowing the platform of the cartridge. The actuator may be a step motor.

According to a further exemplary embodiment, a bioanalytical reaction device is provided, having a reservoir for filling the at least one sample chamber, wherein the reservoir is connectable with the at least one sample chamber over a line ending in a mechanical connection of the actuator with the actuation means for moving the platform. Within the mechanical connection, there also may be the fluidal connection of the bioanalytical reaction device with the cartridge. The fluidal interface or fluidal connection of the bioanalytical reaction device and the mechanical connection are integrated in one component.

According to a further exemplary embodiment, a bioanalytical reaction device is provided having a cartridge presence sensor for detecting the presence and/or the correct insertion of the cartridge in the slot. Only when a cartridge is present in the slot, the bioanalytical reaction device should operate the line for filling the sample chamber. Otherwise, fluids can polute the interior of the bioanalytical reaction device.

According to a further exemplary embodiment, a bioanalytical reaction device is provided, which is adapted to effect the actuator to move the platform in the extended position, when the cartridge presence sensor detects the presence of the cartridge in the slot.

These and other aspects of the invention will be apparent from and elucidated with reference to the embodiment described hereinafter.

### BRIEF DESCRIPTION OF THE DRAWINGS

Below, an embodiment of the present invention is described in more detail with reference to the attached drawings. It shows:
Fig. 1 shows a perspective view of a cartridge for a bioanalytical reaction device with a platform in the stowed position.
Fig. 2 shows a perspective view of the cartridge of Fig. 1 with the platform in an extended position.
Fig. 3 shows a schematic cross sectional view of parts of the platform of Fig. 2.
Fig. 4 is a schematic topview on the platform of Fig. 2.
Fig. 5 shows a schematical diagram of functional components of a bioanalytical reaction device.

### DETAILED DESCRIPTION OF EMBODIMENTS

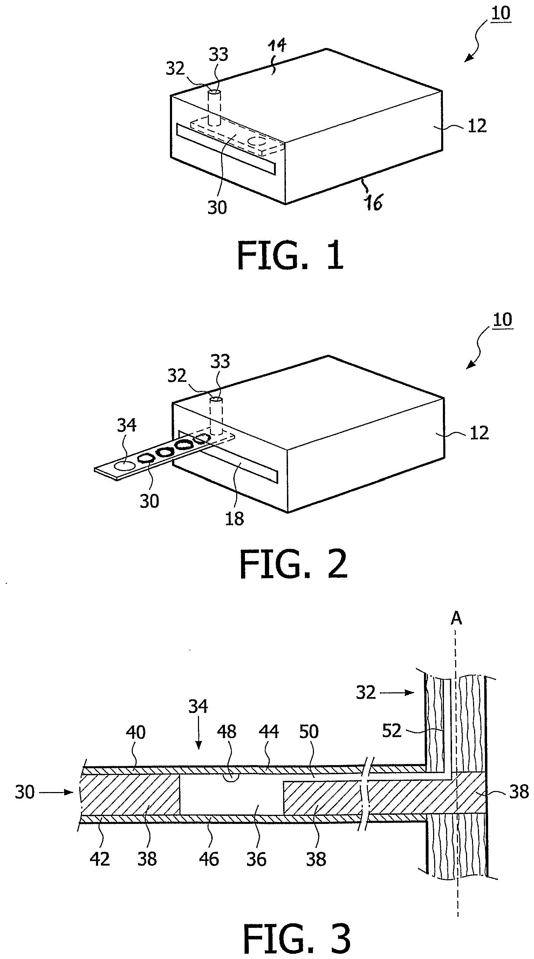

Fig. 1 shows a perspective view of a cartridge 10 for a bioanalytical reaction device. The cartridge 10 has a housing 12 with an upper cover or wall 14 and a lower cover or wall 16. It is to be understood that the wording "upper" and "lower" are used for reasons of simplicity and are not intended to be limiting. For example, the cartridge 10 may be inserted into a bioanalytical reaction device not in the shown orientation but in an upstanding orientation.

Fig. 1 shows the platform 30 in a stowed position. The platform 30 is rotatably connected with the housing 12 via a shaft 32 as actuation means. The shaft 32 is guided by the opening 33 in the upper cover 14. By rotating the shaft 32 about the rotation axis A the platform 30 can be extended from the housing 12 of the cartridge 10.

Fig. 2 shows a perspective view of the cartridge 10 with the platform 30 in an extended position. The platform 30 has exited the housing 12 through a slit 18 in the housing 12 between the upper cover 14 and the lower cover 16. By a further rotation of the shaft 32 in the opposite direction around the rotation axis A, the platform 30 can again be stowed in the housing 12. In the stowed position the platform 30 is protected from being damaged or getting dirty. In the extended position the platform 30 can be accessed by actuators like a heater or a sensor of a bioanalytical reaction device.

Further, in Fig. 2 it can be seen that the platform 30 comprises five sample chambers 34.

Fig. 3 shows a schematic cross-sectional view of parts of the platform 30. In particular, the left-hand side of the drawing shows a cross-sectional view of a sample chamber 34, the right-hand side of the drawing shows a cross-sectional view of the vicinity of the rotation axis A.

Platform 30 comprises a plate 38 that may be made of plastics. For each sample chamber 34 there is an opening 36 in the plate 38. On one first side of the plate 38, a first or upper foil 40 is applied. For example, the upper foil 40 may be glued to the plate 38. In the shown embodiment, the upper foil 40 has a thickness of about 100 µm. In the region of the opening 36 the upper foil 40 forms a thin wall of the sample chamber, the thin wall being a heating interface 44 of the sample chamber 34. If a heating or cooling source is arranged outside of the sample chamber 34 in the region of the heating interface 44 heat may be transferred to the interior of the sample chamber 34 or may exit it.

On the other second side of the plate 38, opposite to the first side, there is applied a second or lower foil 42 of a translucent material. The lower foil 42 may be glued or in some other way be connected to the plate 38. Also, the lower foil 42 has a thickness of about 100 µm. In the region of the opening 36, the lower foil 42 forms an optical interface 46 of the sample chamber 34. In this region, light can penetrate the translucent lower foil 42. Light coming from the interior of the sample chamber can be detected by an optical sensor arranged near the optical interface 46 of the sample chamber 34.

Further, Fig. 3 shows a first channel 48 formed by a groove or notch in the surface of the plate 38 and covered by the upper foil 40. In the same way a second channel 50 is formed connecting the sample chamber 34 with a third channel 52 within the shaft 32.

It is to be understood, that there are other possibilities to form the sample chamber 34 and the channels 48, 50, 52 within the platform 30. For example, the platform 30 may be manufactured from two parts being mirror symmetric and having openings and grooves which form the sample chambers and the channels, when the two parts are connected with each other. Further, it would be possible, to provide the plate 30 with pits. With a foil or thin layer covering the pits sample chambers can be formed on the plate. In this case, such sample chambers would have only one interface.

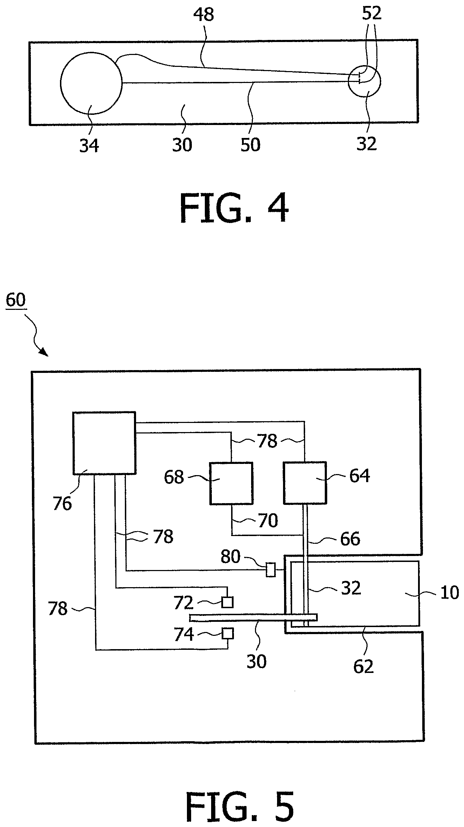

From Fig. 4 being a schematic top view on the platform 30, it can be seen, that the sample chambers 34 are fluidly connected via channels 48, 50 with channels 52 formed in the shaft 32 in the vicinity of the rotation axis A. Over the channels 48 and 50 each sample chamber 34 can be filled with solutions, e.g. a solution containing DNA fragments to be analyzed or amplified. Also, the sample chambers 34 can be emptied by conducting a gas, e.g. air, or other solutions or liquids like water through the channels 48, 50 into the sample chamber 34.

The shaft 32 with the channels 52 is a fluidal interface 54 of the platform 30.

Since the fluidal interface 54 is in the vicinity of the rotation axis A, it can be accessed over the mechanical connection of the bioanalytical reaction device for rotating the platform 30. Therefore, the mechanical connection and the fluidic connection are combined and the number of connections between the cartridge 10 and a bioanalytical reaction device is reduced.

Fig. 5 shows a schematical diagram of a bioanalytical reaction device 60. The bioanalytical reaction device 60 has a slot 62 for receiving the cartridge 10. With an actuator 64, for example a step motor, which is rotatably connected with the shaft 32 the platform 30 can be extended from the cartridge 10 to an extended position and be returned in a stowed position. Fig. 5 shows the platform 30 in an extended position. The fluid lines 70 are connected with inlets and outlets combined with the mechanical connection 66. The inlets and outlets fit to their respective counterparts formed in the shaft 32. A pump and reservoir mechanism 68 can fill the sample chambers 34 in the platform 30. The bioanalytical reaction device has one or more heaters 72 for heating the samples within the sample chambers 34 from the first side of the platform 30 and one or more optical sensors 74 for analyzing the light emitted from the interior of the sample chambers 34 from the second side of the platform 30.

Over a controller 76 which is connected over control lines 78 with the actuator 64, the pump and reservoir mechanism 68, the heater 72 and the optical sensor 74, the bioanalytical reaction device 60 can control the analysis and processing of the samples in the sample chambers in an automated way. For example, the bioanalytical reaction device 60 can conduct the above mentioned PCR procedure.

Further, it is possible, that the bioanalytical reaction device 60 controls the extension and the stowing of the platform 30 in an automated way. When an operator inserts the cartridge 10 into the slot 62, a mechanical sensor 80 detects the presence of the cartridge 10. Alternatively, the detection can be done with an optical sensor. With this input the controller 76 directs the actuator 64 to rotate the platform 30 in the extended position. After that, several processings, like filling the chambers with different solutions, heating the sample chambers 34 and analyzing the light from the sample chambers 34, can be performed by the controller 76. When the processing and the analysis is done, the controller 76 directs the actuator 64 to rotate the platform 30 back to the stowed position and an operator can remove the cartridge 10 from the bioanalytical reaction device 60.

## Claims

1. A cartridge (10) for a bioanalytical reaction device (60), the cartridge (10) comprising:
at least one sample chamber (34) for a sample,
a housing and a platform (30), wherein the platform (30) comprises the at least one sample chamber (34), and wherein the platform (30) is moveably connected to the housing, such that by actuating an actuation means, the platform (30) is moveable between a stowed position, and an extended position,
**characterized in that**
the at least one sample chamber (34) has a wall through which the sample is processed or analyzed by the bioanalytical reaction device (60), and **in that**
the wall is protected by the housing in the stowed position and is outside of the housing in the extended position, and **in that**
the at least one sample chamber (34) is connected to a channel for filling the at least one sample chamber (34), the channel ending in the vicinity of the actuation means, wherein vicinity is understood as relating to a length of one of the following intervals: [0, 15mm], [0, 10mm], [0, 5mm], and **in that**
a part of the channel is located within the actuation means.

2. The cartridge (10) of claim 1, wherein the wall is arranged at a first side of the platform (30), wherein the platform (30) has a second side opposite to the first side, and wherein the platform in the extended position is accessible from the first side and the second side by the bioanalytical reaction device (60) for processing or analyzing the sample.

3. The cartridge (10) of claim 1, wherein at least one dimension of the cartridge (10) with the platform (30) in the extended position is bigger than this dimension of the cartridge (10) with the platform (30) in the stowed position.

4. The cartridge (10) of claim 1, wherein the platform is rotatably connected to the housing.

5. The cartridge (10) of claim 4, wherein the actuation means is a shaft and the platform is connected to the shaft for rotating the platform (30) about a rotation axis.

6. The cartridge (10) of claim 1, wherein the platform is slidably connected to the housing.

7. The cartridge (10) of claim 1, wherein the platform has the form of a plate, which, in the stowed position, is arranged between a first wall and a second wall of the housing.

8. The cartridge (10) of claim 1, wherein the at least one sample chamber (34) is formed by an opening in the platform (30) which is covered by a foil forming the wall.

9. A bioanalytical reaction device (60) having a slot for receiving the cartridge (10) of claim 1, comprising an actuator for extending and stowing the platform (30) of the cartridge (10).

10. The bioanalytical reaction device (60) of claim 9, wherein the actuator is a motor.

11. The bioanalytical reaction device (60) of claim 10 having a reservoir for filling the at least one sample chamber (34), wherein the reservoir is connectable with the at least one sample chamber (34) over a line ending in a mechanical connection of the actuator with the actuation means for moving the platform (30).

12. The bioanalytical reaction device (60) of claim 9 having a cartridge presence sensor for detecting the presence of the cartridge (10) in the slot.

13. The bioanalytical reaction device (60) of claim 12, which is adapted to effect the actuator to move the platform (30) in the extended position, when the cartridge presence sensor detects the presence of the cartridge (10) in the slot.

## Patentansprüche

1. Eine Kartusche (10) für eine bioanalytische Reaktionsvorrichtung (60), wobei die Kartusche (10)
mindestens eine Probenkammer (34) für eine Probe,
ein Gehäuse und eine Plattform (30) umfasst, wobei die Plattform (30) die mindestens eine Probenkammer (34) umfasst, und wobei die Plattform (30) beweglich mit dem Gehäuse verbunden ist, so dass die Plattform (30) zwischen einer Verstauposition und einer ausgefahrenen Position durch Betätigung eines Ansteuerungsmittels bewegbar ist,
**dadurch gekennzeichnet, dass** die mindestens eine Probenkammer (34) eine Wand hat, durch welche die Probe von der bioanalytischen Reaktionsvorrichtung (60) bearbeitet oder analysiert wird, und dass
die Wand in der Verstauposition vom Gehäuse geschützt ist und in der ausgefahrenen Position ausserhalb des Gehäuses ist, und dass
die mindestens eine Probenkammer (34) mit einem Kanal zum Befüllen der mindestens einen Probenkammer (34) verbunden ist, wobei der Kanal in der Nähe des Ansteuerungsmittels endet, wobei die Nähe als bezogen auf eine Länge eines der folgenden Intervalle verstanden wird:
[0, 15 mm], [0, 10 mm], [0, 5 mm], und dass ein Teil des Kanals innerhalb des Ansteuerungsmittels angeordnet ist.

2. Die Kartusche (10) nach Anspruch 1, wobei die Wand an einer ersten Seite der Plattform (30) angeordnet ist, wobei die Plattform (30) gegenüber der ersten Seite eine zweite Seite hat, und wobei die Plattform in der ausgefahrenen Position von der bioanalytischen Reaktionsvorrichtung (60) zur Verarbeitung oder Analyse der Probe von der ersten Seite und von der zweiten Seite zugänglich ist.

3. Die Kartusche (10) nach Anspruch 1, wobei mindestens eine Dimension der Kartusche (10) mit der Plattform (30) in der ausgefahrenen Position grösser als diese Dimension der Kartusche (10) mit der Plattform (30) in der Verstauposition ist.

4. Die Kartusche (10) nach Anspruch 1, wobei die Plattform drehbar mit dem Gehäuse verbunden ist.

5. Die Kartusche (10) nach Anspruch 4, wobei das Ansteuerungsmittel eine Welle ist und die Plattform mit der Welle verbunden ist, um die Plattform (30) um eine Rotationsachse zu drehen.

6. Die Kartusche (10) nach Anspruch 1, wobei die Plattform gleitend mit dem Gehäuse verbunden ist.

7. Die Kartusche (10) nach Anspruch 1, wobei die Plattform die Form einer Platte hat, welche in der Verstauposition zwischen einer ersten Wand und einer zweiten Wand des Gehäuses angeordnet ist.

8. Die Kartusche (10) nach Anspruch 1, wobei die mindestens eine Probenkammer (34) von einer Öffnung in der Plattform (30) gebildet ist, welche von einer die Wand bildenden Folie bedeckt ist.

9. Eine bioanalytische Reaktionsvorrichtung (60), die ein Fach zur Aufnahme der Kartusche (10) nach Anspruch 1 hat, und die einen Antrieb zum Ausfahren und Verstauen der Plattform (30) der Kartusche (10) umfasst.

10. Die bioanalytische Reaktionsvorrichtung (60) nach Anspruch 9, wobei der Antrieb ein Motor ist.

11. Die bioanalytische Reaktionsvorrichtung (60) nach Anspruch 10, die ein Reservoir zum Befüllen der mindestens einen Probenkammer (34) hat, wobei das Reservoir mit der mindestens einen Probenkammer (34) über eine Leitung, die in einer mechanischen Verbindung des Antriebs mit dem Ansteuerungsmittel zum Bewegen der Plattform (30) endet, verbindbar ist.

12. Die bioanalytische Reaktionsvorrichtung (60) nach Anspruch 9, die im Fach einen Sensor für die Anwesenheit der Kartusche zur Detektion der Anwesenheit der Kartusche (10) hat.

13. Die bioanalytische Reaktionsvorrichtung (60) nach Anspruch 12, welche ausgestaltet ist um auf den Antrieb zu wirken, so dass sich die Plattform (30) in die ausgefahrene Position bewegt, wenn der Sensor für die Anwesenheit der Kartusche die Anwesenheit der Kartusche (10) im Fach detektiert.

## Revendications

1. Une cartouche (10) pour un dispositif bioanalytique de réaction (60), la cartouche (10) comprenant:
au moins une chambre d'échantillon (34) pour un échantillon,
une carcasse et une plateforme (30), la plateforme (30) comprenant l'au moins une chambre d'échantillon (34), et la plateforme (30) étant reliée de manière mobile à la carcasse, de sorte que la plateforme (30) est mobile entre une position rentrée et une position étendue en actionnant un moyen d'actionnement,
**caractérisée en ce que** l'au moins une chambre d'échantillon (34) a une paroi à travers laquelle l'échantillon est traité ou analysé par le dispositif bioanalytique de réaction (60), et **en ce que**
la paroi est protégée par la carcasse dans la position rentrée et elle est à l'extérieur de la carcasse dans la position étendue, et **en ce que**
l'au moins une chambre d'échantillon (34) est connectée à un canal pour remplir l'au moins une chambre d'échantillon (34), le canal finissant dans le voisinage du moyen d'actionnement, le voisinage étant considéré comme lié à une longueur d'un des intervalles suivants:
[0, 15 mm], [0, 10 mm], [0, 5 mm], et **en ce qu'**une partie du canal est situé à l'intérieur du moyen d'actionnement.

2. La cartouche (10) selon la revendication 1, la paroi étant arrangée à un premier côté de la plateforme (30), la plateforme (30) ayant un deuxième côté opposé au premier côté, et la plateforme dans la position étendue étant accessible du premier côté et du deuxième côté par le dispositif bioanalytique de réaction (60) afin de traiter ou analyser l'échantillon.

3. La cartouche (10) selon la revendication 1, au moins une dimension de la cartouche (10) avec la plateforme (30) dans la position étendue étant plus grande que cette dimension de la cartouche (10) avec la plateforme (30) dans la position rentrée.

4. La cartouche (10) selon la revendication 1, la plateforme étant connectée avec la carcasse de manière rotative.

5. La cartouche (10) selon la revendication 4, le moyen d'actionnement étant un arbre, et la plateforme étant connectée avec l'arbre afin de tourner la plateforme (30) autour d'un axe de rotation.

6. La cartouche (10) selon la revendication 1, la plateforme étant connectée avec la carcasse de manière glissante.

7. La cartouche (10) selon la revendication 1, la plateforme ayant la forme d'une plaque arrangée entre une première paroi et une deuxième paroi de la carcasse dans la position rentrée.

8. La cartouche (10) selon la revendication 1, l'au moins une chambre d'échantillon (34) étant formée par une ouverture dans la plateforme (30), qui est couverte par une pellicule qui forme la paroi.

9. Un dispositif bioanalytique de réaction (60) ayant un logement pour recevoir la cartouche (10) selon la revendication 1, comprenant un actionneur pour étendre et rentrer la plateforme (30) de la cartouche (10).

10. Le dispositif bioanalytique de réaction (60) selon la revendication 9, l'actionneur étant un moteur.

11. Le dispositif bioanalytique de réaction (60) selon la revendication 10, ayant un réservoir pour remplir l'au moins une chambre d'échantillon (34), le réservoir étant connectable avec l'au moins une chambre d'échantillon (34) via un raccord qui finit dans une connexion mécanique de l'actionneur avec le moyen d'actionnement afin de déplacer la plateforme (30).

12. Le dispositif bioanalytique de réaction (60) selon la revendication 9, ayant un capteur de présence de cartouche pour détecter la présence de la cartouche (10) dans le logement.

13. Le dispositif bioanalytique de réaction (60) selon la revendication 12, qui est adapté à effectuer que l'actionneur déplace la plateforme (30) dans la position étendue quand le capteur de présence de cartouche détecte la présence de la cartouche (10) dans le logement.
